# EUROPÄISCHE PATENTANMELDUNG

(11) **EP 0 574 726 A1**
(43) Veröffentlichungstag der Anmeldung: **22.12.1993**
(21) Anmeldenummer: 93108238.2
(22) Anmeldetag: 21.05.1993
(51) Int. Cl.: C07D 498/04, C07D 271/12, A61K 31/41, A61K 31/435

(54) **Annellierte 1,2,5-Oxadiazol-2-oxide und ihre Verwendung als pharmakologische Wirkstoffe**

(30) Priorität: 10.06.1992 DE 4218977
(71) Anmelder: HOECHST AKTIENGESELLSCHAFT, D-65926 Frankfurt am Main (DE)
(72) Erfinder: Brendel, Joachim, Dr., D-61118 Bad Vilbel (DE); Bohn, Helmut, Dr., W-6369 Schöneck 1 (DE); Schönafinger, Karl, Dr., W-8755 Alzenau (DE)

(57) **Zusammenfassung**

Die vorliegende Erfindung betrifft annellierte 1,2,5-Oxadiazol-2-oxide der allgemeinen Formel I 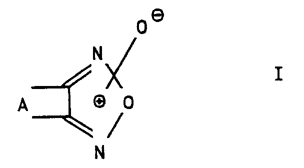
worin
- A: 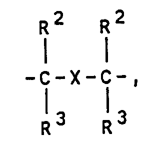 -(CH₂)ₙ-Y-, -CH₂-Z-CO-, 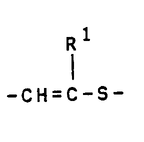 oder 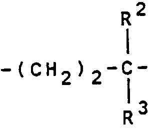 bedeutet;
und X, Y, Z sowie R¹, R², R³ und n wie in Anspruch 1 angegeben definiert sind, Verfahren zu ihrer Herstellung und ihre Verwendung.

## Beschreibung

Die vorliegende Erfindung betrifft annellierte 1,2,5-Oxadiazol-2-oxide, ihre Herstellung und ihre Verwendung als pharmakologische Wirkstoffe.

Eine Reihe von annellierten 1,2,5-Oxadiazol-2-oxiden sind bereits bekannt und beispielsweise in der DE-A 29 12 447 beschrieben.

Die vorliegende Erfindung betrifft annellierte 1,2,5-Oxadiazol-2-oxide der allgemeinen Formel I 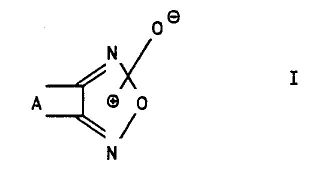
worin
- A: 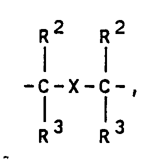 -(CH₂)ₙ-Y-, -CH₂-Z-CO-, 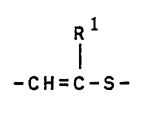 oder 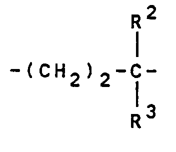 bedeutet;
- n: für 2 oder 3 steht;
- X: -O-, -N(R²)-, -N(COR⁴)-, -S-, -S(O)-, -S(O)₂-, -CH₂- oder -CH₂CH₂- bedeutet;
- Y: -S-, -S(O)-, -S(O)₂-, 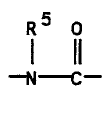 oder 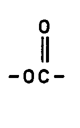 bedeutet;
- Z: -C(R²R³)-, -O-, -S-, N(H)-, N(CH₃)-, 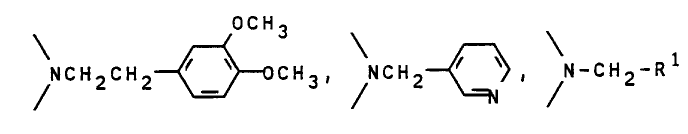 oder -CH₂- bedeutet;
- R¹: -COOH, -COOR⁶ oder -CONR²R³ bedeutet;
- R²: Wasserstoff oder (C₁-C₆)-Alkyl bedeutet;
- R³: (C₁-C₆)-Alkyl bedeutet;
- R⁴: Wasserstoff, (C₁-C₆)-Alkyl, oder gegebenenfalls substituiertes Aryl bedeutet;
- R⁵: Wasserstoff, (C₁-C₆)-Alkyl, (C₂-C₄)-Alkenyl, gegebenenfalls substituiertes Aryl,
-(CH₂)ₚ-R⁷, -CH₂COOR², -CH₂CONR²R³, -CH₂CON B oder
-CH₂)ᵣD bedeutet;
- R⁶: (C₂-C₄)-Alkyl bedeutet;
- R⁷: gegebenenfalls substituiertes Aryl oder Heteroaryl bedeutet;
- B: -(CH₂)_{q}-, -(CH₂)₂-O-(CH₂)₂- oder 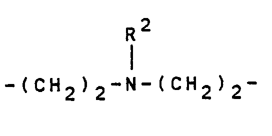 bedeutet;
- D: -OH, -OR² oder -NR²R³ bedeutet; sowie
- p: für 1, 2 oder 3;
- q: für 4, 5 oder 6; und
- r: für 2, 3 oder 4 steht,
sowie deren pharmakologisch annehmbare Säureadditionsverbindungen.

Für R², R³, R⁴, R⁵ oder R⁶ stehende Alkylreste können geradkettig oder verzweigt sein. Beispiele für solche Alkylreste sind Methyl, Ethyl, n-Propyl, i-Propyl, n-Butyl, i-Butyl, Pentyl und Hexyl.

Für R⁵ stehendes (C₂-C₄)-Alkenyl kann ebenfalls geradkettig oder verzweigt sein. Beispiele sind Vinyl und Allyl.

Für R⁴, R⁵ oder R⁷ stehendes Aryl ist insbesondere (C₆-C₁₄)-Aryl, wobei Phenyl bevorzugt ist.

Für R⁷ stehendes Heteroaryl ist bevorzugt 5- bis 7-gliedrig und leitet sich beispielsweise von Pyrrol oder Pyridin ab. Bevorzugt sind α-Pyridyl und ß-Pyridyl.

Für R⁴, R⁵ oder R⁷ stehendes Aryl und Heteroaryl können auch substituiert sein. Geeignete Substituenten sind beispielsweise (C₁-C₄)-Alkyl, (C₁-C₄)-Alkoxy, Amino, (C₁-C₄)-Alkylamino, Di-(C₁-C₄)-alkylamino, (C₁-C₆)-Alkanoylamino, Halogen, vorzugsweise Fluor, Chlor oder Brom, Hydroxy, Nitro oder Cyano. Aryl oder Heteroaryl können durch die genannten Substituenten auch mehrfach, beispielsweise zwei- oder dreifach substituiert sein.

Ein bevorzugter substituierter Arylrest ist 3,4-Dimethoxyphenyl.

Bevorzugt sind ferner:
- n: 2
- R²: Wasserstoff, Methyl
- R³: Methyl, Ethyl
- R⁴: Methyl, Phenyl
- R⁶: Ethyl
- R⁷: Phenyl, 3,4-Dimethoxyphenyl, 2-Pyridyl, 3-Pyridyl
- p: 1, 2
- q: 4, 5
- r: 2, 3
Besonders bevorzugt sind:
- X: -O-, -N(H)-, -N(CH₃)-, N(CO-Phenyl)-, -N(COCH₃)-, -CH₂-, -CH₂CH₂-
- Y: -S(O)₂-, 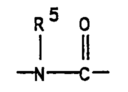
- Z: -N(H)-, -N(CH₃)-, 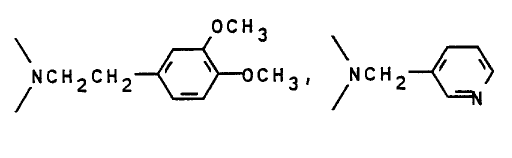 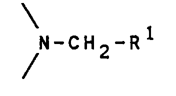
- R¹: -COOH, -COOC₂H₅, -COONHCH₃
- R⁵: Wasserstoff, -CH₃, -C₂H₅, -Phenyl, -(CH₂)ₚ-R⁷,
-CH₂COOR², -CH₂CONR²R³, -CH₂CON B, -(CH₂)ᵣD
Die erfindungsgemäßen Verbindungen der allgemeinen Formel I können dadurch hergestellt werden, daß 1,2-Dioxime der allgemeinen Formel II 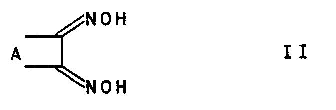
worin A wie oben angegeben definiert ist, oxidiert werden.

Als Oxidationsmittel für diese an sich bekannte Reaktion können Hypohalogenite wie Natrium- oder Kaliumhypochlorit oder -bromit, Metallsalze wie Kaliumhexacyanoferrat (III) oder Bleitetraacetat, nitrose Gase wie NO₂ oder Iodosoverbindungen wie z.B. Bis-(trifluoracetoxy)-iodbenzol eingesetzt werden.

Aus Dioximen der allgemeinen Formel II mit unsymmetrischem Rest A können in Abhängigkeit von der Konfiguration der eingesetzten Oxime und den angewendeten Reaktionsbedingungen zwei Isomere der allgemeinen Formel I mit unterschiedlicher Lage der N-Oxid-Funktion, d.h. 1-Oxid- oder 3-Oxid-Verbindungen, entstehen.

Verbindungen der allgemeinen Formel I mit A = -(CH₂)ₙY- oder -CH₂-Z-CO- lassen sich durch Erhitzen auf 80 bis 140^{o}C in einem inerten Lösungsmittel, wie beispielsweise Toluol oder Petrolether, ineinander umlagern. Aus den so erhaltenen Gleichgewichtsgemischen können die einzelnen Isomere durch übliche Trennverfahren, wie beispielsweise Kristallisation oder Chromatographie als reine Verbindungen isoliert werden.

Für die Anwendungen der Verbindungen der allgemeinen Formel I ist die Trennung der Isomeren jedoch nicht zwingend erforderlich, so daß sie auch unterbleiben kann.

Bei Verbindungen der allgemeinen Formel I mit A = -CH=CR¹-S- ist eine solche Trennung nicht möglich, weil sich diese Isomere bereits bei Raumtemperatur rasch ineinander umwandeln.

Die vorliegende Erfindung betrifft demnach auch Isomerengemische von annellierten 1,2,5-Oxadiazol-2-oxiden der allgemeinen Formel I, worin
- A: -(CH₂)ₙ-Y-, -CH₂-Z-CO-, -CH=CR¹-S oder -(CH₂)₂CR²R³-bedeutet
und Y, Z, R¹, R², R³ und n wie oben angegeben definiert sind.

Die 1,2-Dioxime der allgemeinen Formel II können entweder aus 1,2-Dionen der allgemeinen Formel III 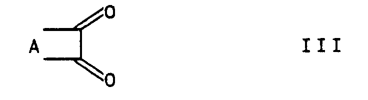
oder aus 1,2-Dionmonooximen der allgemeinen Formel IV 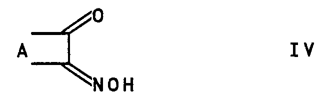
durch Umsetzung mit überschüssigem Hydroxylamin erhalten werden (siehe z.B. Houben-Weyl, Methoden der organischen Chemie, Band X/4, Seite 55 bis 77).

Die Verbindungen der allgemeinen Formeln III und IV sind entweder bekannt oder lassen sich nach bekannten Methoden herstellen.

Beispielsweise können Verbindungen der allgemeinen Formel V 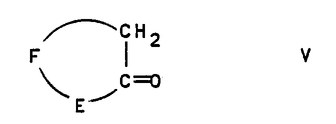
worin
- E: -C(R²R³)-, 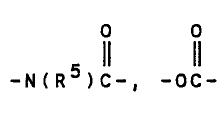 oder 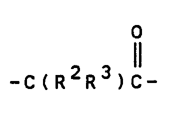
bedeutet, F so gewählt ist, daß E und F zusammen A entsprechend obiger Definitionen ergeben und R², R³ und R⁵ wie oben angegeben definiert sind, durch Nitrosierung der Methylengruppe, beispielsweise mit Natriumnitrit in Salz- oder Essigsäure (siehe z.B. H. v. Dobeneck et al., Liebigs Ann. Chem. 1976, 476) oder mit Amylnitrit in Gegenwart eines Alkalimetallalkoholats (siehe z.B. C. Sandris, G. Ourisson, Bull. Soc. Chim. Fr. 1958, 345, Houben-Weyl, Band X/4, Seite 17 - 44), in Dionmonoxime der allgemeinen Formel IV oder alternativ durch Oxidation, beispielsweise mit Selendioxid (siehe z.B. C. Sandris, G. Ourisson, Bull. Soc. Chim. Fr. 1958, 345, Houben-Weyl, Band 4/1a, Seite 351 - 353), in Dione der allgemeinen Formel III überführt werden.
Alternativ können aber auch Verbindungen der allgemeinen Formel VI 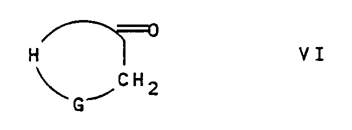
worin
- G: 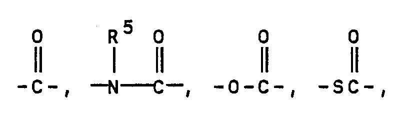 -S-, -S(O) oder
-S(O)₂ bedeutet, H so gewählt wird, daß G und H zusammen A entsprechend obiger Definitionen ergeben und R⁵ wie oben angegeben definiert ist, durch Reaktion mit Natriumnitrit in wäßriger Salzsäure nitrosiert werden. Man erhält dadurch ebenfalls Verbindungen der allgemeinen Formel IV (siehe z.B. J. Ackrell, A. J. Boulton, J. Chem. Soc. Perkin Trans. I 1973, 351).

Die Herstellung von Verbindungen der allgemeinen Formel VI ist bekannt und beispielsweise in EP-A 149 534, J. Antibiotics 33 (1980), 173 und J. Chem. Soc. (C) 1967, 2171 beschrieben.

Für die Synthese von Verbindungen der allgemeinen Formel I mit A = -CH=C(R¹)-S- ist es bevorzugt, eine Verbindung der allgemeinen Formel VII 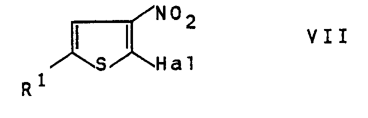
worin Hal Chlor oder Brom bedeutet und R¹ wie oben angegeben definiert ist, mit Natriumazid in erfindungsgemäße Verbindungen der allgemeinen Formel Ia 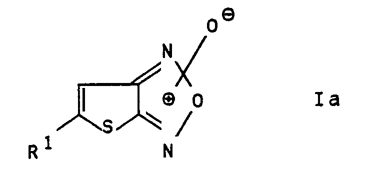
zu überführen (siehe z.B. J. Chem. Soc. Perkin Trans II, 1989, 127).

Gemäß den vorstehenden Herstellungsverfahren können neben den in den Beispielen beschriebenen, auch die folgenden erfindungsgemäßen Verbindungen hergestellt werden:
5-Phenyl-6,7-dihydro[1,2,5]oxadiazolo-[3,4-c]pyridin-4-(5H)-on-1-oxid und -3-oxid, sowie die entsprechenden 5-(3-Pyridylmethyl)-, 5-Methyl-, 5-Ethyl-, 5-Propyl-, 5-Isoproypl-, 5-Butyl-, 5-Hexyl-, 5-Allyl-, 5-(3,4-Dimethoxybenzyl)-, 5-(3,4-Dimethoxyphenylethyl)-, 5-(N,N-Diethylaminoethyl)-, 5-(N,N-Dimethylaminocarbonylmethyl)- und 5-Hydroxyethylverbindungen;
6,7-Dihydro[1,2,5]oxadiazolo[3,4-c]pyridin-4(5H)-on-1-oxid und -3-oxid;
5,6,7,8-Tetrahydro[1,2,5]oxadiazolo-[3,4-c]azepin-4-on-1-oxid und -3-oxid;
5-Benzyl-5,6,7,8-tetrahydro[1,2,5]oxadiazolo-[3,4-c]azepin-4-on-1-oxid und -3-oxid, sowie die entsprechenden 5-(3-Pyridylmethyl)-Verbindungen;
5,6-Dihydro-thieno[2,3-c][1,2,5]oxadiazoll-oxid und -3-oxid sowie die entsprechenden 4-oxo-Derivate;
6,7-Dihydro[1,2,5]oxadiazolo[3,4-c]pyran-4-on-1-oxid und -3-oxid;
5-Methyl-5,6-dihydro-4,4,6,6-tetramethyl-4H-pyrrolo-[3,4-c][1,2,5]oxadiazol-1-oxid sowie die entsprechende 5-Acetyl-Verbindung;
4,5,6,7-Tetrahydro-4,4,7,7-tetramethylbenzofurazan-1-oxid;
4,5,6,7-Tetrahydro-4,7-dimethylbenzofurazan-1-oxid;
5,6-Dihydro-cyclopent[c][1,2,5]oxadiazol-4(4H)-on-1-oxid und -3-oxid;
5,6-Dihydro-5,5-dimethyl-cyclopent[c][1,2,5]oxadiazol-4(4H)-on-1-oxid und -3-oxid;
Thieno[2,3-c][1,2,5]oxadiazol-5-carbonsäure-1-oxid und -3-oxid sowie die entsprechenden 5-Carbonsäureethylamid-Verbindungen;
4-Methyl- und 6-Methyl-5,6-dihydro-4H-cyclopent [c]-[1,2,5]oxadiazol-1-oxid sowie die entsprechenden 4,4-Diethyl- und 6,6-Diethyl-Verbindungen;
4H, 6H-4,6-Dimethylfuro[3,4-c][1,2,5]oxadiazol-1-oxid;
4H, 6H-Furo[3,4-c][1,2,5]oxadiazol-4-on-1-oxid und -3-oxid sowie die entsprechenden 4H, 6H-Thieno-Verbindungen;
5,6-Dihydro-5-(3-pyridylmethyl)-4H-pyrrolo[3,4-c][1,2,5]-oxadiazol-4-on-1-oxid und -3-oxid sowie die entsprechenden 5-Hydroxycarbonylmethylverbindungen.

Erfindungsgemäße Verbindungen der allgemeinen Formel I, die eine basische Gruppe enthalten, können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel I und ihre pharmakologisch annehmbaren Säureadditionssalze besitzen wertvolle pharmakologische Eigenschaften. Am Modell der Kalium-depolarisierten Pulmonalarterie des Meerschweinchen führen sie in niedrigen Konzentrationen zu einer Relaxation. Diese Wirkung kann mit Oxyhämoglobin inhibiert werden, was auf einen NO-mediierten Mechanismus deutet.

Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatiorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die Verbindungen der allgemeinen Formel I ihre pharmakologisch annehmbaren Säureadditionssalze sowie ihre Isomerengemische können somit bei den genannten Indikationen eingesetzt werden. Vor allem aber haben sich NO-Donoren zur Behandlung und Prophylaxe von angina pectoris bewährt.

Die Verbindungen der allgemeinen Formel I und ihre pharmakologisch annehmbaren Säureadditionssalze sowie Isomerengemische können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon oder eines Isomerengemisches, neben üblichen pharmazeutisch einwandfreien Träger- und Zusatzstoffen enthalten.

Die Heilmittel können oral, z.B. in Form von pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: ß-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I, ihre pharmakologisch annehmbaren Säureadditionsssalze, ihre Isomerengemische und pharmazeutische Präparate, welche die Verbindungen der allgemeinen Formel I oder ihre pharmakologisch annehmbaren Säureadditionssalze oder ein Isomerengemisch als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüber hinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

### Beispiel 1:

### 5,6-Dihydro-4,4-dioxothieno[2,3-c][1,2,5]oxadiazol-1-oxid und -3-oxid

a) Zu einer Suspension von 15,5 g (116 mmol) 3-Oxotetrahydrothiophendioxid (M.A. Smith et al., J. Chem. Soc. (c) 1967, 2171) in 70 ml Wasser und 10 ml konz. Salzsäure wird bei 0^{o}C eine Lösung von 8,4 g (120 mmol) Natriumnitrit in 25 ml Wasser zugetropft. Nach 1 Stunde bei 0^{o}C wird das Produkt abgesaugt, mit Wasser gewaschen und im Vakuum getrocknet. Man erhält 11,5 g (61 %) 2-Hydroxyimino-3-oxotetrahydrothiophendioxid, Fp. 148^{o}C.
b) Zu einer Lösung von 11,4 g (70 mmol) 2-Hydroxyimino-3-oxotetrahydrothiophendioxid in 100 ml Methanol werden 5,4 g (77 mmol) Hydroxylaminhydrochlorid gelöst in 15 ml Wasser zugegeben. Nach 2 Stunden bei Raumtemperatur wird der Ansatz vollständig eingeengt und der Rückstand aus Ethanol umkristallisiert. Man erhält 6,2 g (50 %) 2,3-Dihydroxyiminotetrahydrothiophendioxid, Fp. 181^{o}C.
c) Zu einer Suspension von 1,5 g (8,4 mmol) 2,3-Dihydoxyiminotetrahydrothiophendioxid in 250 ml CH₂Cl₂ wird eine Lösung von 4,4 g (10 mmol) Bis(trifluoracetoxy)-iodbenzol in 280 ml CH₂Cl₂ zugetropft und 1 Stunde bei Raumtemperatur gerührt. Aus dem vollständig eingeengten Ansatz kristallisiert das Produkt über Nacht teilweise aus. Nach Verrühren mit Isopropanol erhält man 1,0 g (68 %) 5,6-Dihydro-4,4-dioxo-thieno[2,3-c][1,2,5]oxadiazol-1-oxid und -3-oxid im Verhältnis 1 : 1 (NMR), Fp. 76^{o}C.

### Beispiel 2:

### 4H, 6H-4,4,6,6-Tetramethylfuro[3,4-c][1,2,5]oxadiazol-1-oxid

a) 10,0 g (54 mmol) käufliches 2,2,5,5-Tetramethyl-3,4-(2H,5H)-furandionhydrazonoxim werden in 800 ml Wasser mit 75 g (1,1 mmol) Hydroxylaminhydrochlorid 2 Stunden unter Rückfluß erhitzt. Das ausgefallene Produkt wird abgesaugt und mit Wasser gewaschen. Man erhält 8 g (86 %) 2,2,5,5-Tetramethyl-3,4(2H,5H)-furandiondioxim als Gemisch von 2 Isomeren, Fp. 252^{o}C.
b) Zu einer Lösung von 3,0 g (16 mmol) 2,2,5,5-Tetramethyl-3,4(2H,5H)-furandiondioxim in 15 ml 10 %ige Natronlauge werden 90 ml 14 %ige Natriumhypochloritlösung bei 0^{o}C rasch zugegeben. Der ausgefallene Niederschlag wird sofort abgesaugt, mit Wasser gewaschen und aus Isopropanol umkristallisiert. Man erhält 2,0 g (67 %) 4H,6H-4,4,-6,6-Tetramethylfuro[3,4-c][1,2,5]oxadiazol-1-oxid, Fp. 121^{o}C.

### Beispiel 3:

### 4-Ethyl- und 6-Ethyl-5,6-dihydro-4H-cyclopent[c][1,2,5]-oxadiazol-1-oxid

a) Zu einer Lösung von 14 g (200 mmol) Hydroxylaminhydrochlorid und 16,5 g (200 mmol) Natriumacetat in 150 ml Wasser wird bei 60^{o}C eine Lösung von 5,0 g (40 mmol) 3-Ethyl-2-hydroxy-2-cyclopenten-1-on in 5 g 1,2-Propandiol zugetropft. Nach 2 Stunden bei 70^{o}C wird der ausgefallene Niederschlag abgesaugt und mit Wasser gewaschen. Man erhält 5,0 g (81 %) 3-Ethylcyclopentan-1,2-diondioxim.
b) Zu einer Lösung von 5,0 g (32 mmol) 3-Ethylcyclopentan-1,2-diondioxim in 200 ml 10 %iger Natronlauge werden 200 ml 14 %ige Natriumhypochloritlösung bei 0^{o}C zugetropft. Nach Extrahieren mit Essigester und Destillieren im Vakuum bei 104^{o}C/0,1 Torr erhält man 3,0 g (60 %) 4-Ethyl- und 6-Ethyl-5,6-dihydro-4H-cyclopent[c][1,2,5]-oxadiazol-1-oxid im Verhältnis 1 : 1 (NMR).

### Beispiel 4:

### 5-Benzoyl-5,6-dihydro-4,4,6,6-tetramethyl-4H-pyrrolo-[3,4-c][1,2,5]oxadiazol-1-oxid.

a) Eine Lösung von 0,7 g (2,7 mmol) 1-Benzoyl-2,2,5,5-tetramethylpyrrolidin-3,4-dion (C. Sandris, G. Ourisson, Bull. Soc. Chim. Fr. 1958, 345) und 1,1 g (16 mmol) Hydroxylaminhydrochlorid in 60 ml Ethanol/Wasser (1 : 1) wird 10 Stunden auf 60^{o}C erhitzt. Der ausgefallene Niederschlag wird abgesaugt, mit Wasser gewaschen und im Vakuum getrocknet. Man erhält 0,65 g (83 %) 1-Benzoyl-2,2,5,5-tetramethyl-3,4-pyrrolidindiondioxim, Fp. 235 - 240^{o}C.
b) 0,55 g (1,9 mmol) 1-Benzoyl-2,2,5,5-tetramethyl-3,4-pyrrolidindiondioxim werden analog Beispiel 2b umgesetzt und man erhält 0,36 g (66 %) 5-Benzoyl-5,6-dihydro-4,4,-6,6-tetramethyl-4H-pyrrolo[3,4-c][1,2,5]oxadiazol-1-oxid, Fp. 147 - 149^{o}C.

### Beispiel 5:

### 5,6-Dihydro-4,4,6,6-tetramethyl-4H-pyrrolo[3,4-c][1,2,5]-oxadiazol-1-oxid.

Aus 3,1 g (18 mmol) 2,2,5,5-Tetramethylpyrrolidin-3,4-dion-3-oxim werden analog Beispiel 4 1,3 g (45 %) 5,6-Dihydro-4,4,6,6-tetramethyl-4H-pyrrolo[3,4-c][1,2,5]oxadiazol-1-oxid erhalten, ¹³C-NMR (DMSO): δ = 25,0 (q), 27,5 (q), 66,7 (s), 67,7 (s), 115,6 (s), 164,4 (s), Fp. (Hydrochlorid): 184 - 186^{o}C.

### Beispiel 6:

### 5,6-Dihydro-5-methyl-4H-pyrrolo[3,4-c][1,2,5]oxadiazol--4-on-1-oxid und -3-oxid.

a) Zu einer Lösung von 17,0 g (0,15 mol) 1-Methyl-2,3-pyrrolidindion (H. Rapoport et al., J. Org. Chem. 40 (1975) 1264) in 40 ml Eisessig und 30 ml Chloroform werden bei 0^{o}C 10,5 g (0,15 mol) Natriumnitrit in 50 ml Wasser zugetropft. Man rührt noch 2 Stunden, filtriert das ausgefallene Produkt ab und erhält 12,0 g (58 %) 1-Methyl-2,3,4-pyrrolidintrion-4-oxim.
b) 9,0 g (63 mmol) 1-Methyl-2,3,4-pyrrolidintrion-4-oxim werden mit 15 g Hydroxylaminhydrochlorid und 15 g Natriumacetat 2 Stunden auf 95^{o}C erhitzt, und man erhält 8,5 g (86 %) 1-Methyl-2,3,4-pyrrolidintrion-3,4-dioxim.
c) Zu einer Suspension von 1,0 g (6,4 mmol) 1-Methyl-2,3,4-pyrrolidintrion-3,4-dioxim in 50 ml Essigester wird eine Lösung von 13,0 g (38 mmol) Kaliumhexacyanoferrat (III) in 40 ml Wasser und 3 ml gesättigter Sodalösung zugetropft, und es wird 2 Stunden bei Raumtemperatur gerührt. Die organische Phase wird abgetrennt, eingeengt und der Rückstand durch Flash-Chromatographie (Essigester/Cyclohexan 60 : 40) gereinigt. Man erhält 0,34 g (34 %) 5,6-Dihydro-5-methyl-4H-pyrrolo[3,4-c][1,2,5]oxadlazol-4-on-1-oxid und -3-oxid im Verhältnis 1 : 2 (NMR), Fp. 49 - 52^{o}C.

### Beispiel 7:

### Analog Beispiel 6 wurden 5,6-Dihydro-5-(3,4-dimethoxyphenylethyl)-4H-pyrrolo[3,4-c][1,2,5]oxadiazol-4-on-1-oxid und -3-oxid erhalten (Isomere im Verhältnis 1 : 4), Fp. 165^{o}C.

### Beispiel 8:

### 5-Benzyl-6,7-dihydro[1,2,5]oxadiazolo[3,4-c]pyridin-4(5H)-on-1-oxid.

a) Zu einer Lösung von 7,0 g (34,5 mmol) 1-Benzylpiperidin-2,4-dion (S. Takano et al., Tetrahedron Lett. 1979, 369) in 30 ml Essigester und 30 ml Wasser werden bei 0^{o}C 7 ml konzentrierte Salzsäure zugesetzt und dann 2,5 g (36 mmol) Natriumnitrit in 10 ml Wasser zugetropft. Nach 1 Stunde bei 0^{o}C wird der ausgefallene Niederschlag abgesaugt, und man erhält 6,6 g (85 %) 1-Benzyl-piperidin-2,3,4-trion-3-oxim, Fp. 121 - 122^{o}C.
b) Aus 4,6 g 1-Benzyl-piperidin-2,3,4-trion-3-oxim werden analog Beispiel 1b 3,4 g (68 %) 1-Benzyl-piperidin-2,3,4-trion-3,4-dioxim, Fp. 170 - 172^{o}C, erhalten.
c) Zu einer Lösung von 3,0 g (12 mmol) 1-Benzylpiperindin-2,3,4-trion-3,4-dioxim in 80 ml 10 %iger Natronlauge werden 80 ml 14 %ige Natriumhypochloritlösung bei 0^{o}C rasch zugetropft. Der ausgefallene Niederschlag wird sofort abgesaugt, mit Wasser gewaschen und im Vakuum getocknet. Man erhält 2,6 g (87 %) 5-Benzyl-6,7-dihydro [1,2,5]oxadiazolo[3,4-c]pyridin-4(5H)-on-1-oxid, Fp. 118 - 120^{o}C, ¹H-NMR (DMSO) : δ = 2,95 (t), 3,65 (t), 4,70 (t), 7,35 (s), ¹³C-NMR (DMSO) : δ = 18,0 (t), 44,8 (t), 49,2 (t), 111,9 (s), 127,4 (d), 127,7 (d), 128,5 (d), 136,2 (s), 149,6 (s), 155,7 (s).

### Beispiel 9:

### 5-Benzyl-6,7-dihydro[1,2,5]oxadiazolo[3,4-c]-pyridin-4(5H)-on-3-oxid.

1,6 g 5-Benzyl-6,7-dihydro[1,2,5]oxadiazolo[3,4-c]-pyridin-4(5H)-on-1-oxid werden in 200 ml Toluol 4 Stunden unter Rückfluß erhitzt. Das Toluol wird abgezogen und der Rückstand aus Isopropanol umkristallisiert. Man erhält 1,4 g 5-Benzyl-6,7-dihydro[1,2,5]oxadiazolo[3,4-c]-pyridin-4(5H)-on-3-oxid und -1-oxid im Verhältnis 4 : 1, Fp. 112 -114^{o}C, ¹H-NMR (DMSO) : δ = 3,10 (t), 3,65 (t), 4,65 (s), 7,35 (s).

### Beispiel 10:

### Thieno[2,3-c][1,2,5]oxadiazol-5-carbonsäureethylester-1-oxid und -3-oxid.

Eine Suspension von 25,0 g (90 mmol) 2-Brom-3-nitro-5-thiophencarbonsäureethylester (Tetrahedron 21 (1965) 1061) und 29,0 g (450 mmol) Natriumazid in 300 ml Methanol wird 4 Stunden bei Raumtemperatur gerührt. Man gießt auf 4 l Eiswasser, filtriert ab und erhitzt den Rückstand in 700 ml Toluol 3 Stunden auf 70^{o}C. Anschließende Reinigung durch Flash-Chromatographie liefert 6,5 g (34 %) Thieno[2,3-c][1,2,5]oxadiazol-5-carbonsäureethylester-1-oxid und -3-oxid, Fp. 72 - 74^{o}C.

## Patentansprüche

1. Annellierte 1,2,5-Oxadiazol-2-oxide der allgemeinen Formel I 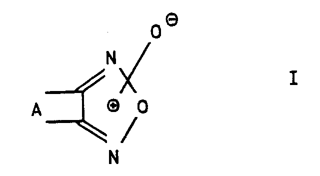 worin
A 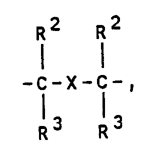 -(CH₂)ₙ-Y-, -CH₂-Z-CO-, 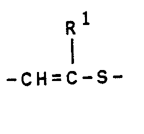 oder 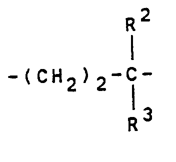 bedeutet;
n für 2 oder 3 steht;
X -O-, -N(R²)-, -N(COR⁴)-, -S-, -S(O)-, -S(O)₂-, -CH₂- oder -CH₂CH₂- bedeutet;
Y -S-, -S(O)-, -S(O)₂-, 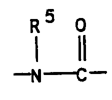 oder 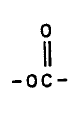 bedeutet;
Z -C(R²R³)-, -O-, -S-, -N(H)-, -N(CH₃)-, 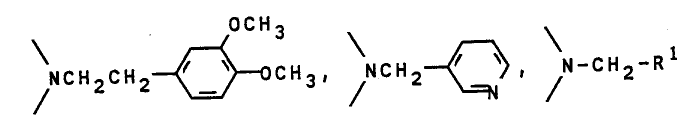 oder -CH₂- bedeutet;
R¹ -COOH, -COOR⁶ oder -CONR²R³ bedeutet;
R² Wasserstoff oder (C₁-C₆)-Alkyl bedeutet;
R³ (C₁-C₆)-Alkyl bedeutet;
R⁴ Wasserstoff, (c₁-c₆)-Alkyl, oder gegebenenfalls substituiertes Aryl bedeutet;
R⁵ Wasserstoff, (C₁-C₆)-Alkyl, (C₂-C₄)-Alkenyl, gegebenenfalls substituiertes Aryl,
-(CH₂)ₚ-R⁷, -CH₂COOR², -CH₂CONR²R³, -CH₂CON B oder
-(CH₂)ᵣD bedeutet;
R⁶ (C₂-C₄)-Alkyl bedeutet;
R⁷ gegebenenfalls substituiertes Aryl oder Heteroaryl bedeutet;
B -(CH₂)_{q}-, -(CH₂)₂-O-(CH₂)₂- oder 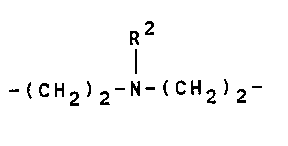 bedeutet;
D -OH, -OR² oder -NR²R³ bedeutet; sowie
p für 1, 2 oder 3;
q für 4, 5 oder 6; und
r für 2, 3 oder 4 steht,
sowie deren pharmakologisch annehmbare Säureadditionsverbindungen.

2. Annellierte 1,2,5-Oxadiazol-2-oxide der allgemeinen Formel I nach Anspruch 1, dadurch gekennzeichnet, daß
n 2
R² Wasserstoff, Methyl
R³ Methyl, Ethyl
R⁴ Methyl, Phenyl
R⁶ Ethyl
R⁷ Phenyl, 3,4-Dimethoxyphenyl, 2-Pyridyl, 3-Pyridyl
p 1, 2
q 4, 5
r 2, 3
bedeuten.

3. Annellierte 1,2,5-Oxadiazol-2-oxide der allgemeinen Formel I nach Anspruch 1 und/oder 2, dadurch gekennzeichnet, daß
X -O-, -N(H)-, -N(CH₃)-, N(CO-Phenyl)-, -N(COCH₃)-, -CH₂-, -CH₂CH₂-
Y -S(O)₂-, 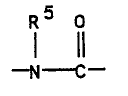
Z -N(H)-, -N(CH₃)-, 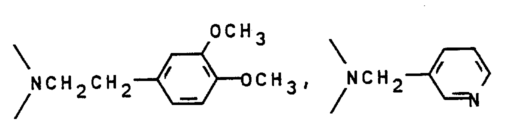 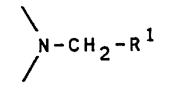
R¹ -COOH, -COOC₂H₅, -COONHCH₃
R⁵ Wasserstoff, -CH₃, -C₂H₅, -Phenyl, -(CH₂)ₚ-R⁷,
-CH₂COOR², -CH₂CONR²R³, -CH₂CON B, -(CH₂)ᵣD
bedeuten.

4. Isomerengemische von annellierten 1,2,5-Oxadiazol-2-oxiden der allgemeinen Formel I gemäß einem oder mehreren dar Ansprüche 1 bis 3, worin A -(CH₂)ₙ-Y-, -CH₂-Z-CO-, -CH=CR¹-S- oder -(CH₂)₂CR²R³ bedeutet und Y, Z, R¹, R², R³ und n wie in Anspruch 1 angegeben definiert sind.

5. Verfahren zur Herstellung von annellierten 1,2,5-oxadiazol-2-oxiden der allgemeinen Formel I gemaß Anspruch 1, dadurch gekennzeichnet, daß 1,2-Dioxime der allgemeinen Formel II 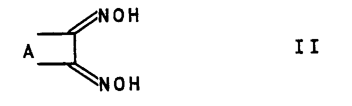 worin A wie in Anspruch 1 angegeben definiert ist, oxidiert werden.

6. Verfahren zur Herstellung von Verbindungen der allgemeinen Formel Ia 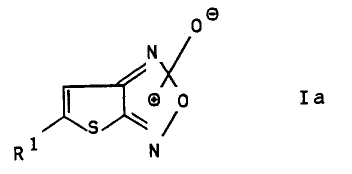 worin R¹ wie in Anspruch 1 angegeben definiert ist, dadurch gekennzeichnet, daß eine Verbindung der allgemeinen Formel VII 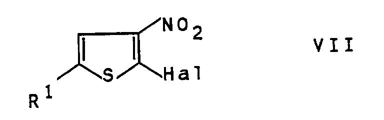 worin Hal Chlor oder Brom bedeutet, mit Natriumazid umgesetzt wird.

7. Verwendung von annellierten 1,2,5-Oxadiazol-2-oxiden der allgemeinen Formel I gemäß einem oder mehreren der Ansprüche 1 bis 3 zur Bekämpfung und Vorbeugung von Erkrankungen des kardiovaskulären Systems.

8. Verwendung von annellierten 1,2,5-Oxadiazol-2-oxiden der allgemeinen Formel I gemäß einem oder mehreren der Ansprüche 1 bis 3 zur Bekämpfung und Vorbeugung von Angina pectoris.

9. Verwendung von annellierten 1,2,5-Oxadiazol-2-oxiden der allgemeinen Formel I gemäß einem oder mehreren der Ansprüche 1 bis 3 zur Behandlung erektiler Dysfunktionen.

10. Pharmazeutisches Präparat, dadurch gekennzeichnet, daß es ein annelliertes 1,2,5-Oxadiazol-2-oxid der allgemeinen Formel I gemäß einem oder mehreren der Ansprüche 1 bis 3, ein pharmakologisch annehmbares Säureadditionssalz davon oder ein Isomerengemisch gemäß Anspruch 4 als Wirkstoff zusammen mit pharmazeutisch annehmbaren Träger- und Zusatzstoffen und gegebenenfalls noch ein oder mehrere andere pharmakologische Wirkstoffe enthält.
